(19) **Europäisches Patentamt / European Patent Office / Office européen des brevets**

(11) **EP 0 913 432 B1**

(12) **EUROPEAN PATENT SPECIFICATION**

(45) Date of publication and mention
of the grant of the patent:
**28.09.2011 Bulletin 2011/39**

(21) Application number: **97925293.9**

(22) Date of filing: **06.06.1997**

(51) Int Cl.:
*C09C 3/00* (2006.01)      *C08K 9/00* (2006.01)
*C09D 11/00* (2006.01)      *C09K 3/00* (2006.01)
*C10M 125/04* (2006.01)     *C10M 171/06* (2006.01)

(86) International application number:
**PCT/JP1997/001939**

(87) International publication number:
**WO 1997/047693 (18.12.1997 Gazette 1997/54)**

(54) **MULTILAYER COATED POWDER**

MEHRSCHICHTIG BESCHICHTETES PULVER

POUDRE REVETUE DE COUCHES MULTIPLES

(84) Designated Contracting States:
**AT BE CH DE DK ES FI FR GB GR IE IT LI LU MC NL PT SE**

(30) Priority: **10.06.1996   JP 14741796**
        **10.06.1996   JP 14741896**
        **10.06.1996   JP 14742196**

(43) Date of publication of application:
**06.05.1999   Bulletin 1999/18**

(73) Proprietors:
• **NITTETSU MINING CO., LTD.**
  **Tokyo 100 (JP)**
• **Nakatsuka, Katsuto**
  **Sendai-shi-**
  **Miyagi 982-02 (JP)**

(72) Inventors:
• **NAKATSUKA, Katsuto**
  **Sendai-shi,**
  **Miyagi 982-02 (JP)**

• **ATARASHI, Takafumi,**
  **Nittetsu Mining Co., Ltd.**
  **Nishitama-gun,**
  **Tokyo 190-0182 (JP)**

(74) Representative: **Grünecker, Kinkeldey, Stockmair & Schwanhäusser Anwaltssozietät**
  **Leopoldstrasse 4**
  **80802 München (DE)**

(56) References cited:
**EP-A- 0 515 928      EP-A- 0 668 329**
**GB-A- 2 253 839      JP-A- 1 006 093**
**JP-A- 1 158 077      JP-A- 2 016 044**
**JP-A- 2 029 472      JP-A- 3 093 898**
**JP-A- 3 120 351      JP-A- 4 168 163**
**JP-A- 7 207 251      JP-A- 8 302 237**
**JP-A- 58 177 911     JP-A- 60 169 412**
**JP-B- 44 003 083**

**Description**

**TECHNICAL FIELD**

The present invention relates to a multilayer-coated powder and a pigment and material for a cosmetic. More particularly, this invention relates to a pigment powder for use in a color ink and as a filler for plastics/papers; and a material for color cosmetics which comprises a lightweight powder.

**BACKGROUND ART**

The present inventors previously invented a powder comprising a base particle of a metal or metal compound and having thereon a metal oxide film which has an even thickness from 0.01 to 20 $\mu$m and in which the metal contained therein is different from the metal constituting the base particle in order to provide a powder which combines properties inherent only in the metal particle or metal compound particle with other properties so as to have a combination of functions (Unexamined Published Japanese Patent Application No. 6-228604). The present inventors further improved the above powder and invented a powder comprising a base particle having not a single layer of a metal oxide but plural layers of a metal oxide film and a metal film alternatively (Unexamined Published Japanese Patent Application No. 7-90310).

For producing these powders, it is necessary to form plural metal oxide films each having an even thickness over a base particle. Since it is difficult to deposit a metal oxide or a metal compound as a precursor therefrom an aqueous metal salt solution, the present inventors developed a method which comprises dispersing the base particle into a metal alkoxide solution and hydrolyzing the metal alkoxide to thereby form a metal oxide film on the base particle. Due to this method, it has become possible to form a thin metal oxide film which is even in thickness and, in particular, the formation of a multilayered metal oxide film has become possible.

Attempts were made to use these multilayer-coated powders as powders combining properties inherent only in given a base particle with other properties so as to have a combination of functions, mainly in applications, such as pigments, and materials for cosmetics.

However, for use in inks, the powders comprising a metal or metal compound as a base have a problem that the powder particles are apt to precipitate in the liquid before use because of their large specific gravity and are difficult to disperse evenly. For use as a powder for inks, a filler for plastics, and the like, the powders should be colored. However, since use of a mechanochemical method results in pulverization of the pigment itself and, hence, in a reduced pigment particle diameter and a lighter color, it is necessary to use a dye or the like to color the powders. There also is a problem that when the powders are used as a filler for plastics and subjected to kneading so as to be dispersed into a resin, then powder particles separate from the shells formed by a mechanochemical method and this may result in a color change.

With respect to materials for cosmetics, in particular, materials for emulsion-form cosmetics, there is a problem that the powders are difficult to use. In the case of a powder obtained by depositing particles of titanium oxide or the like on the particle surfaces of, for example, a resin powder, there is a problem that powder particles separate from the surface coating shells during use to cause a color change.

JP-A-60-169412 discloses a cosmetic having a coated mica compounded therein, the mica being prepared by coating the surface of mica with a low-order titanium oxide or titanium nitride oxide or a titanium compound containing at least one of low-order titanium oxide and titanium nitride oxide, and further coating the coated surface of the mica with a titanium compound whose surface is coated with titanium oxide.

JP-A-2-16044 discloses a thin film structure comprising a lower layer and a multilayered interference coating, which is supported by the lower layer and produces an inherent color shift with an angle, wherein the interference coating has a metal-dielectric structure containing a metal having magnetic properties and refractive properties.

JP-A-3-120351 discloses an additive for a plastic molding obtained by powdering or fragmenting a base material coated with an interference coating film having a thickness, which can show an objective color by interference of light.

JP-A-4-168163 discloses a design coating composition comprising an interference pearl mica pigment comprising mica and an inorganic thin film layer formed on the surface of the mica, and a metal-plated mica pigment having a bright layer and formed by plating a metal on the surface of the interference pearl mica pigment.

JP-A-8-302237 discloses a goniochromatic luster pigment, which is based on a multiply coated platelet-shaped metallic substrate comprising at least one layer packet of (A) a layer composed mainly of silicon oxide, silicon oxide hydrate, aluminum oxide, and/or aluminum oxide hydrate, and (B) a metallic layer, which is composed mainly of aluminum and is at least partially transparent to visible light, and also, if desired, additionally (C) an outer layer, which is composed mainly of a colorless or selectively absorbing metal oxide, and/or contains a phosphate, a chromate, and/or a vanadate.

JP-B-44-3083 discloses an iris pearl pigment comprising a transparent thin plate-like sandwich foil comprising two transparent thin plates (nd ≤ 1,600) made of a high-refractive index substance (n ≥ 1.8) and an interlayer (nd = 1,000 to 20,000) comprising substances having a refractive index of at least 0.2 lower than that of the high-refractive index substance, wherein the interlayer is formed and intimately adhered between the two thin plates.

JP-A-58-177911 discloses a cosmetic comprising a transparent cosmetic base having thereon a plate polymer in the form of many layer.

JP-A-1-158077 discloses a color mica titanium layer having thereon (i) a titanium oxide basic layer containing dark color regions; and (ii) a color tone adjusting layer comprising (a) at least one of oxides of silicon, aluminum, and zinc, or (b) a composite oxide containing at least two elements selected from silicon, aluminum, and zinc.

JP-A-3-93898 discloses an electrically viscous fluid comprising fine particles in an electrically insulating liquid, wherein the fine particles are prepared by forming an electrically conductive layer on the surfaces of electrically insulating fine particles, and further forming an electrically insulating coating film on the surfaces of the fine particles.

JP-A-1-6093 discloses an electrical viscous fluid obtained by dispersing (A) an oily medium having electrical insulation characteristics with (B) dielectric fine particles consisting of electrically conductive particles coated with an electrical insulation thin film.

JP-A-2-29472 discloses an ink composition obtained by dispersing or suspending dielectrically polarizable polymer particles which can be dyed with a reactive dye in an insulating liquid having a specific electrical resistance.

GB-A-2253839 discloses an inorganic titanate powder having a first coating layer of silicon oxide, and a second coating layer of tin oxide containing antimony oxide.

EP-A-668329 discloses an interference pigment, comprising a plate-like metallic core having provided thereon a multilayer-coating.

JP-A-07-207251 discloses a cosmetic comprising pigment particles containing a silicon oxide core having on the surface thereof a multilayer-coating.

EP-A-515928 discloses a pigment comprising a plate-like titanium oxide core having provided on the surface thereof a first iron oxide layer, and a further polymer layer.

An object of the present invention is to provide: a pigment powder of a stable color tone which comprises a lightweight base particle and is capable of being colored without using a dye or pigment and with which not only a pigment for monochromatic color inks, e.g., blue, green, or yellow, inks, can be obtained by dispersing the pigment powder into a fluid, but also a filler for plastics/papers can be designed; and a material for color cosmetics which is a powder with which monochromatic colored powders, such as mascaras or eyebrow pencils, can be designed and which is capable of absorbing ultraviolet and infrared rays harmful to the skin.

DISCLOSURE OF THE INVENTION

The present inventors made intensive studies. As a result, they have found that a powder colored in a stable tone, such as blue, green, or yellow, can be obtained without a dye or a pigment by forming a thin film comprising plural layers differing in refractive index on the surface of a powder to regulate the multilayered film with respect to reflected-light interference waveform. The present invention has thus been completed.

The present invention provides a multilayer-coated powder comprising a base particle having a specific gravity of 0.1 to 10.5 and having thereon plural coating layers which are different from each other in refractive index, wherein the base particle is a spherical particle, and wherein each unit of the coating layers has an interference reflection peak or an interference transmission bottom at the same specific wavelength, wherein the thickness of each unit of the coating layer is determined by fixing a fundamental film thickness thereof which satisfies the following equation (1):

$$N \times d = m \times \lambda/4 \qquad (1)$$

(wherein N represents a complex refractive index, d represents the fundamental film thickness, m represents an integer (natural number), and λ represents the wavelength at which the interference reflection peak or interference transmission peak appears, and N is defined by the following equation (2):

$$N = n + i\kappa \qquad (2)$$

(wherein n represents the refractive index of each unit coating layer, i represents complex number, and κ represents extinction coefficient)), and correcting the actual thickness of the each unit of the coating layers based on the function of the phase shift caused by the extinction coefficient κ of refractive index, the phase shift occurring at film interfaces, and the peak shift attributable to refractive index dispersion and particle shape so that the each unit of the coating layers has an interference reflection peak or an interference transmission bottom at the same specific wavelength.

The invention further provides a pigment powder comprising said multilayer-coated powder.

Moreover, the invention provides a material for a cosmetic comprising said multilayer-coated powder.

Preferred embodiments of the present invention are set forth in the sub-claims.

In the present invention, the base particle having a specific gravity from 0.1 to 10.5, which constitutes the base of the multilayer-coated powder, is not particularly limited and may be made of either an organic or an inorganic substance. The specific gravity of this base is preferably from 0.1 to 5.5, more preferably from 0.1 to 2.8, from the standpoints of flowability and suspensibility. If the base has a specific gravity below 0.1, the buoyancy of the base is so large that the film should be made to comprise a larger number of layers or to be exceedingly thick, which is uneconomical. On the other hand, if the base has a specific gravity exceeding 10.5, a thick film for suspending the base is necessary, which also is uneconomical. In addition, in the case of cosmetics and the like, such a powder having too large a particle diameter is unsuitable because it gives a sandy feeling.

For use in a pigment powder or a powder as a material for cosmetics, the base is preferably an inorganic substance because it is not influenced by the heat of burning in the step of coating film formation which will be described later.

Examples of the resin particles include cellulose powders, cellulose acetate powders, and spherical or pulverized particles obtained from polyamides, epoxy resins, polyesters, melamine resins, polyurethanes, vinyl acetate resins, and silicone resins or obtained by the polymerization or copolymerization of acrylic esters, methacrylic esters, styrene, ethylene, propylene, and derivatives thereof. Especially preferred resin particles are spherical acrylic resin particles obtained by the polymerization of acrylic acid or a methacrylic ester.

Usable as the inorganic substance are inorganic hollow particles, such as Shirasu balloons (hollow silicic acid particles), fine hollow carbon particles (Kureca Sphere), fused alumina bubbles, aerosil, white carbon, fine hollow silica spheres, fine hollow calcium carbonate spheres, calcium carbonate, pearlite, talc, bentonite, kaolin, and the like.

In the multilayer-coated powder of the present invention, the plural coating layers formed on each base particle having a specific gravity from 0.1 to 10.5 are different from each other in refractive index. The materials constituting these coating layers are desirably selected suitably from inorganic metal compounds, metals, alloys, and organic substances.

These coating layers each is formed as a dense and continuous film, whereby they can have excellent properties.

Typical examples of the inorganic metal compounds which may constitute the coating layers include metal oxides. Specific examples thereof include the oxides of iron, nickel, chromium, titanium, aluminum, silicon, calcium, magnesium, and barium and composite oxides of these metals, such as barium titanate and lead titanate. Examples of the metal compounds other than metal oxides include metal fluorides, such as magnesium fluoride and calcium fluoride; metal nitrides, such as iron nitride; metal sulfides, such as zinc sulfide and cadmium sulfide; metal carbonates, such as calcium carbonate; metal phosphates, such as calcium phosphate; and metal carbides. In particular, silica or the like enables the multilayer-coated powder to have satisfactory properties.

Examples of the elemental metals which may constitute the coating layers include silver metal, cobalt metal, nickel metal, and iron metal. Examples of the metal alloys include iron-nickel alloys, iron-cobalt alloys, iron-nickel alloy nitrides, and iron-nickel-cobalt alloy nitrides.

The organic substances which may constitute the coating layers may be the same as or different from the aforementioned organic substances usable for constituting the base, and are not particularly limited. However, resins are preferred. Examples of the resins include cellulose, cellulose acetate, polyamides, epoxy resins, polyesters, melamine resins, polyurethanes, vinyl acetate resins, silicone resins, and polymers or copolymers of acrylic esters, methacrylic esters, styrene, ethylene, propylene, and derivatives thereof.

Although various materials can be used to constitute the coating layers as described above, a suitable combination of materials should be selected according to the kind of a pigment or coating material, purposes, the substance to be coated, etc., while taking account of the refractive index of each coating layer.

If the powder is for use as a material for cosmetics, the coating layer as the outermost layer should, of course, be constituted of a material which is inert to the living body or which at least does not adversely influence the living body. Typical examples of such materials include titanium dioxide.

Titanium dioxide is though to be effective also because it has the property of specifically absorbing ultraviolet waves. By using a metal film and a titanium oxide film and suitably regulating their film thicknesses, a powder having a high reflectance throughout the whole infrared region can be obtained and a UV- and IR-cutting cosmetic can be prepared therefrom.

If the coating layers contain one or more organic substance layers, the other layers each is preferably a metal oxide film.

The particle diameter of the multilayer-coated powder of the present invention is not particularly limited, and can be suitably regulated according to purposes. However, the particle diameter thereof is generally in the range from 0.01 $\mu$m to several millimeters. If the powder is to be used for coloring as a coloring material based on visible light interference, the particle diameter thereof is preferably regulated to 0.06 to 1,000 $\mu$m.

The unit coating layers constituting the plural coating layers are ones whose thicknesses have been determined so that these layers have interference reflection peaks or interference transmission bottoms at the same specific wavelength. The thickness of each unit of the coating layer is determined by fixing a fundamental film thickness thereof which satisfies the following equation (1) :

$$N \times d = m \times \lambda/4 \qquad (1)$$

(wherein N represents a complex refractive index, d represents the fundamental film thickness, m represents an integer (natural number), and $\lambda$ represents the wavelength at which the interference reflection peak or interference transmission peak appears, and N is defined by the following equation (2):

$$N = n + i\kappa \qquad (2)$$

(wherein n represents the refractive index of each unit coating layer, i represents complex number, and $\kappa$ represents extinction coefficient)), and correcting the actual thickness of the each unit of the coating layers based on the function of the phase shift caused by the extinction coefficient $\kappa$ of refractive index, the phase shift occurring at film interfaces, and the peak shift attributable to refractive index dispersion and particle shape so that the each unit of the coating layers has an interference reflection peak or an interference transmission bottom at the same specific wavelength as shown above.

For forming these films, the following methods may be used according to the substances to be deposited. However, other methods can be used.

(1) Formation of Organic Substance Film (Resin Film):

a. Polymerization in Liquid Phase

Use can be made, for example, of a method in which particles serving as a base are dispersed and emulsion polymerization is conducted to form a resin film on each particle.

b. Film Formation in Vapor Phase (CVD) (PVD)

(2) Formation of Inorganic Metal Compound Film:

a. Solid Deposition in Liquid Phase

A preferred method is to disperse particles serving as a base into a metal alkoxide solution and hydrolyze the metal alkoxide to hereby form a metal oxide film on each particle. This method can form a dense metal oxide film. It is also possible to react an aqueous solution of a metal salt to thereby form a film of a metal oxide or the like on particles.

b. Film Formation in Vapor Phase (CVD) (PVD)

(3) Formation of Metal Film or Alloy Film:

a. Reduction of Metal Salt in Liquid Phase

Use is made of the so-called chemical plating method in which the metal salt contained in an aqueous metal salt solution is reduced to deposit the metal to thereby form a metal film.

b. Film Formation in Vapor Phase (CVD) (PVD)

A metal film can be formed on the surfaces of particles, for example, by the vapor deposition of a metal.
A method for forming a multilayered film composed of layers of a metal oxide having a high refractive index and, alternately arranged therewith, layers of a metal oxide having a low refractive index is explained below in detail as an example. First, a base particle are dispersed into an alcohol solution of an alkoxide of titanium, zirconium, etc. A mixed solution comprising water, an alcohol, and a catalyst is added dropwise to the dispersion under stirring to hydrolyze the alkoxide to thereby form on the surface of each base particle a film of titanium oxide or zirconium oxide as a high-refractive-index film. Thereafter, this powder is taken out by solid/liquid separation, dried, and then subjected to a heat treatment. The drying may be conducted by any means selected from vacuum drying with heating, vacuum drying, and natural drying. It is also possible to use an apparatus, such as a spray dryer, in an inert atmosphere while regulating the atmosphere. The heat treatment may be accomplished by heating the powder at 150 to 1100°C (when the base particle is an inorganic particle) or at 150 to 500°C (when the base particle is not an inorganic particle) for 1 minute to 3 hours either in air when the coating composition does not undergo oxidation or in an inert atmosphere when the coating composition is susceptible to oxidation. Subsequently, the particles having the high-refractive-index film formed thereon are dispersed into an alcohol solution of a metal alkoxide which gives an oxide having a low refractive index, such as a silicon alkoxide or aluminum alkoxide. A mixed solution comprising water, an alcohol, and a catalyst is added dropwise to the resultant dispersion under stirring to hydrolyze the alkoxide to thereby form over the surface of each base particle a film of silicon oxide or aluminum oxide as a low-refractive-index film. Thereafter, the powder is taken out by solid/liquid separation, vacuum-dried, and then heat-treated in the same manner as the above. As a result of the above procedure, a powder is obtained in which the base particle has, on the surface thereof, two layers comprising a high-refractive-index metal oxide film and a low-refractive-index metal oxide film. Furthermore, the above procedure for forming metal oxide films is repeated to thereby obtain a powder in which each particle has multiple metal oxide films on its surface. Since the powder thus obtained has high-refractive-index metal oxide films alternately arranged with low-refractive-index metal oxide films as stated hereinabove, it has a high reflectance and high whiteness.

For forming a metal film on a base particle or on a metal oxide film, contact electroplating or sputtering may be used, besides the electroless plating described above. However, the contact electroplating has a drawback that powder particles not in contact with an electrode are not plated, while the sputtering has a drawback that a metal vapor is not evenly applied to the powder particles. Namely, the thickness of the coating formed by either method varies from particle to particle. In contrast, the method of film formation by electroless plating is preferred in that a dense and even film can be formed and the film thickness is easy to regulate. The metal film is preferably subjected to a heat treatment after film formation in the same manner as for the metal oxide films.

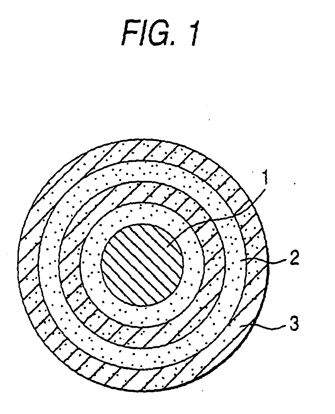

The present invention will be explained below in more detail by reference to the drawings. Fig. 1 is a sectional view diagrammatically illustrating the structure of a particle of a multilayer-coated powder according to the present invention. The particle comprises a base particle 1 having a specific gravity from 0.1 to 10.5 as a nucleus and, alternately formed on the surface thereof, two coating layers 2 and two coating layers 3 differing in refractive index from the layers 2.

A special function can be imparted by regulating the thicknesses of the coating films differing in refractive index alternately formed on the surface of each base particle. Coating films differing in refractive index are alternately formed on each base particle so as to satisfy the following equation (1). Namely, films which each is made of a substance having a refractive index n and has a thickness d corresponding to m (integer) times the value which is one-fourth a wavelength of visible light are formed in an appropriate thickness and number. As a result, the light having a specific wavelength $\lambda$ (the light utilizing Fresnel's interference reflection) is reflected or absorbed.

$$nd \; = \; m\lambda/4 \qquad\qquad (1)$$

This function is utilized as follows. An oxide film having such a thickness and refractive index as to satisfy equation (1) with respect to a target wavelength of visible light is formed on the surface of each base particle, and this film is coated with an oxide film having a different refractive index. This procedure is conducted once or repeated one or more times to thereby form films which have a characteristic reflection or absorption wavelength width in the visible light region. In the above procedure, the sequence of material deposition for film formation is determined in the following manner. When the base has a high refractive index, a film having a low refractive index is preferably formed as the first layer. In the reverse case, a film having a high refractive index is preferably formed as the first layer.

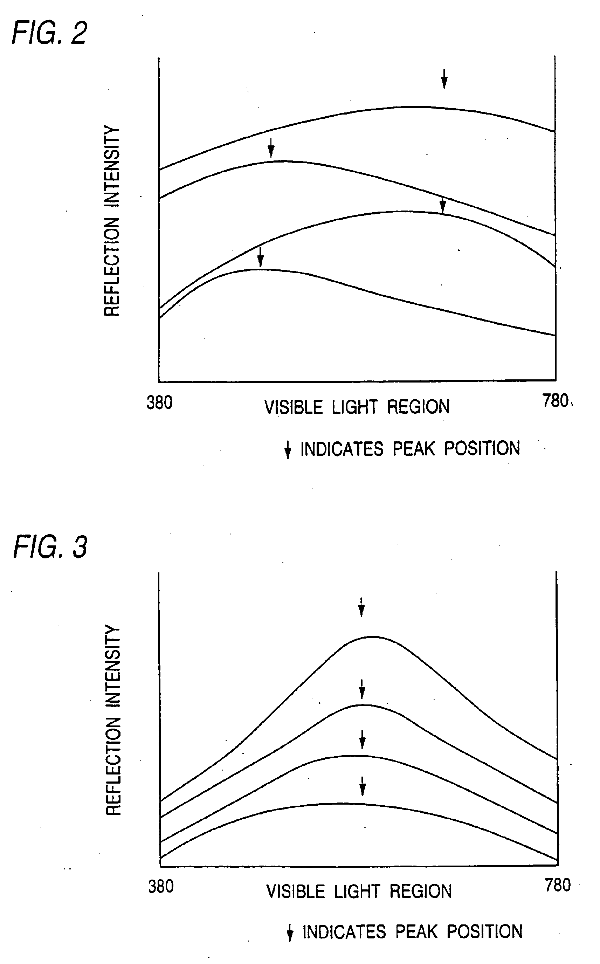

Film thickness is controlled based on a measurement in which the change of optical film thickness, which is the product of the refractive index of the film and the film thickness, is determined as flection waveform with a spectrophotometer or the like. The thickness of each layer is designed so that the reflection waveform conforms to the finally required waveform. For example, if the unit coating films constituting a multilayered film have reflection waveform peaks at different positions as shown in Fig. 2, the powder is white. On the other hand, when the unit coating films are regulated so that the reflection waveform peaks thereof are in exactly the same position as shown in Fig. 3, a monochromatic colored powder, e.g., a blue, green, or yellow powder, can be obtained without using a dye or pigment.

However, in the case of an actual powder, a design should be made while taking account of the particle diameter and shape of the powder, the phase shift occurring at interfaces between film materials and the base particle material, the peak shift attributable to the wavelength dependence of refractive index, etc. If the thickness of the film formed on the base particle is easily explained, the Fresnel interference caused by parallel film formed on a parallel plane is designed under the conditions including the above equation (1) in which n has been replaced with N defined by the following equation (2). Even if a parallel film is formed on a parallel plane, extinction coefficient $\kappa$ is included in the refractive index N of the metal defined by equation (2) even though the particle shape is a plane parallel plate shape. In the case of transparent oxides (dielectrics), $\kappa$ is exceedingly small and negligible.

$$N = n + i\kappa \qquad\qquad (2)$$

(i represents a complex number)

When the extinction coefficient $\kappa$ is large, an enhanced phase shift occurs at the interface between the film material and the base particle material, and this phase shift influences the optimum interference thicknesses of all layers of the multilayered film.

Because of the above, the mere regulation of geometrical film thicknesses results in different peak positions and, hence, in a lighter color especially in monochromatic coloring. In order to avoid this, a design is made beforehand through a computer simulation so as to result in an optimal combination of film thicknesses while taking account of influences of the phase shift on all films.

There also are the phase shift caused by an oxide layer present on a metal surface and the peak shift attributable to the wavelength dependence of refractive index. In order to correct these, it is necessary to use a spectrophotometer or the like to find optimal conditions under which reflection peaks or absorption bottoms appear at target wavelengths in a final target number of films.

In a film formed on a curved surface such as that of a spherical particle, interference occurs similarly to that on plane plates and is basically in accordance with Fresnel's interference principle. Consequently, a coloring method can be designed so as to produce a white powder and a monochromatic powder as shown in Figs. 2 and 3. However, in the case of curved surfaces, the light which has struck on the powder and has been reflected causes complicated interference. The resultant interference waveforms are almost the same as on plane plates when the number of films is small. However, as the total number of films increases, the interference within the multilayered film becomes more complicated. In the case of a multilayered film also, a spectral reflection curve can be designed beforehand based on Fresnel interference through a computer simulation so as to result in an optimal combination of film thicknesses. In particular, if coating films are formed on the surface of each base particle, the influences of a phase shift on the base particle surface and on all films are taken in account when a design is made beforehand through a computer simulation so as to result in an optimal combination of film thicknesses. Furthermore, the peak shift caused by an oxide layer present on the base particle surface and the peak shift attributable to the wavelength dependence of refractive index are also taken in account. In the actual production of a sample, designed spectral curves are referred to and, in order to correct these in actual films, it is necessary to use a spectrophotometer or the like, while changing film thicknesses, to find optimal conditions under which reflection peaks or absorption bottoms appear at target wavelengths in a final target number of films. Also if a powder having irregular particle shapes is colored, interference occurs due to the multilayered film. A basic film design is hence made with reference to conditions for an interference multilayered film for spherical particles. The peak position for each of unit coating films constituting the multilayered film can be regulated by changing the thickness of the layer, and the film thickness can be regulated by changing the solution composition, reaction time, and the number of starting-material addition times. Thus, the powder can be colored in a desired tint. As described above, white and monochromatic powders can be obtained by finding optimal conditions under which reflection peaks or absorption bottoms appear at target wavelengths in a final target number of films, while changing film-forming conditions, such as solutions for film formation. Furthermore, by controlling a combination of materials for forming a multilayered film and the thicknesses of the unit coating films, the color development by interference in the multilayered film can be regulated. Thus, a powder can be colored in a desired bright tint without using a dye or pigment.

BRIEF DESCRIPTION OF THE DRAWINGS

Fig. 1 is a sectional view diagrammatically illustrating the structure of a particle of a multilayer-coated powder according to the present invention; numeral 1 denotes a base particle, 2 a coating layer, and 3 a coating layer.
Fig. 2 is a graphic presentation showing reflection intensity spectral waveforms for the unit coating films constituting the multilayered film of a powder colored in white.
Fig. 3 is a graphic presentation showing reflection intensity spectral waveforms for the unit coating films constituting the multilayered film of a powder colored monochromatically.

BEST MODES FOR CARRYING OUT THE INVENTION

The present invention will be explained below in more detail by reference to Examples.

**EXAMPLE 1**

Intended use: pigment powder

First layer: titania coating

To 10 g of an acrylic powder (average particle diameter, 1.5 $\mu$m; specific gravity, 1.4) was added 250 ml of ethanol to disperse the particles. The container was heated with an oil bath to keep the temperature of the liquid at 55°C. Thereto was added 3.5 g of titanium isopropoxide. This mixture was stirred. A solution prepared by mixing 30 ml of ethanol with 3.5 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-coated powder $A_1$ was obtained. The titania-coated powder $A_1$ obtained had satisfactory dispersibility and was composed of independent particles. This powder $A_1$ had a spectral reflection curve having a peak wavelength of 455 nm and had a reflectance at the peak wavelength of 32%. It was pale-blue.

Second layer: silica coating

To 10 g of the titania-coated powder $A_1$ was added 100 ml of ethanol to disperse the particles. The container was heated with an oil bath to keep the temperature of the liquid at 55°C. Thereto were added 6 g of silicon ethoxide, ammonia water (29%), and 8 g of water. This mixture was allowed to react for 3 hours under stirring. After the reaction, the reaction mixture was diluted and washed with a sufficient amount of ethanol and then filtered. The solid matter obtained was dried with a vacuum dryer at 180°C for 8 hours. After the drying, silica-titania-coated powder $A_2$ was obtained. The silica-titania-coated powder $A_2$ obtained had excellent dispersibility.

Third layer: titania coating

To 10 g of the silica-titania-coated powder $A_2$ was added 250 ml of ethanol to disperse the particles. The container was heated with an oil bath to keep the temperature of the liquid at 55°C. Thereto was added 3.4 g of titanium isopropoxide. This mixture was stirred. A solution prepared by mixing 30 ml of ethanol with 3.4 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-silica-coated powder A was obtained. The titania-silica-coated powder A obtained had satisfactory dispersibility and was composed of independent particles. This powder A had a spectral reflection curve having a peak wavelength of 448 nm and had a reflectance at the peak wavelength of 45%. It was blue.
The refractive index and film thickness of each of the first to third layers are shown in Table 1.

Table 1

| Coating Layers | Refractive Index | Film Thickness (nm) |
| --- | --- | --- |
| First layer: titania film | 2.3 | 50 |
| Second layer: silica film | 1.45 | 99 |
| Third layer: titania film | 2.3 | 49 |

**EXAMPLE 2**

Intended use: pigment powder

First layer: titania coating

A hundred grams of an acrylic powder (average particle diameter, 55 $\mu$m; specific gravity, 1.5) was placed in a lowly vacuum rotary mixing vessel in which a tungsten crucible containing a sufficient amount of a titania powder had been disposed beforehand in an upper part thereof. The crucible was heated to thereby vaporize the titania while stirring the acrylic powder. Thus, the powder in the rotary mixing vessel was coated with titania to obtain titania-coated powder $B_1$. The titania-coated powder $B_1$ obtained had satisfactory dispersibility and was composed of independent particles. This powder $B_1$ had a spectral reflection curve having a peak wavelength of 545 nm and had a reflectance at the peak wavelength of 78%. It was pale-green.

Second layer: magnesium fluoride coating

Eighty grams of the titania-coated powder $B_1$ was likewise placed in a lowly vacuum rotary mixing vessel in which a tungsten crucible containing a sufficient amount of a magnesium fluoride powder had been disposed beforehand in an upper part thereof. The crucible was heated to thereby vaporize the magnesium fluoride while stirring the powder $B_1$. Thus, the powder in the rotary mixing vessel was coated with magnesium fluoride to obtain magnesium fluoride-titania-coated powder $B_2$. The magnesium fluoride-titania-coated powder $B_2$ obtained had satisfactory dispersibility and was composed of independent particles.

Third layer: titania coating

A hundred grams of the magnesium fluoride-titania-coated powder $B_2$ was placed in a lowly vacuum rotary mixing vessel in which a tungsten crucible containing a sufficient amount of a titania powder had been disposed beforehand in an upper part thereof. The crucible was heated to thereby vaporize the titania while stirring the powder $B_2$. Thus, the powder in the rotary mixing vessel was coated with titania to obtain titania/magnesium fluoride-coated powder B. The titania/magnesium fluoride-coated powder B obtained had satisfactory dispersibility and was composed of independent particles. This powder C had a spectral reflection curve having a peak wavelength of 500 nm and had a reflectance at the peak wavelength of 88%. It was blue-green.

The refractive index and film thickness of each of the first to third layers are shown in Table 2.

Table 2

| Coating Layers | Refractive Index | Film Thickness (nm) |
|---|---|---|
| First layer: titania film | 2.6 | 52 |
| Second layer: magnesium fluoride film | 1.38 | 101 |
| Third layer: titania film | 2.6 | 48 |

## EXAMPLE 3

Intended use: powder for cosmetic material

First layer: titania coating

To 10 g of an acrylic powder (average particle diameter, 1.5 $\mu$m; specific gravity, 1.4) was added 250 ml of ethanol to disperse the particles. The container was heated with an oil bath to keep the temperature of the liquid at 55°C. Thereto was added 3.5 g of titanium isopropoxide. This mixture was stirred. A solution prepared by mixing 30 ml of ethanol with 3.5 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-coated powder $C_1$ was obtained. The titania-coated powder $C_1$ obtained had satisfactory dispersibility and was composed of independent particles. This powder had a spectral reflection curve having a peak wavelength of 455 nm and had a reflectance at the peak wavelength of 32%. It was pale-blue.

Second layer: polystyrene coating

To 600 g of distilled water was added 100 g of styrene monomer. While this mixture was heated to 70°C under stirring, sodium lauryl sulfate was added thereto to emulsify the monomer. With this emulsion was mixed 25 g of the titania-coated powder $C_1$. The resultant mixture was agitated at a high speed to sufficiently mix the ingredients. An aqueous ammonium persulfate solution was added thereto in an amount of 10% to initiate a polymerization reaction. The mixture was allowed to react for 4 hours under stirring. After completion of the reaction, the reaction mixture was diluted with 2 liters of distilled water, and the supernatant was discarded by decantation to collect the precipitate. This precipitate was dried on a filter paper to obtain polystyrene-titania-coated powder $C_2$. The polystyrene-titania-coated powder $C_2$ obtained had satisfactory dispersibility and was composed of independent particles.

Third layer: titania coating

To 10 g of the polystyrene-titania-coated powder $C_2$ was added 250 ml of ethanol to disperse the particles. The container was heated with an oil bath to keep the temperature of the liquid at 55°C. Thereto was added 3.4 g of titanium isopropoxide. This mixture was stirred. A solution prepared by mixing 30 ml of ethanol with 3.4 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-polystyrene-coated powder C was obtained. The titania-polystyrene-coated powder C obtained had satisfactory dispersibility and was composed of independent particles. This powder C had a spectral reflection curve having a peak wavelength of 448 nm and had a reflectance at the peak wavelength of 45%. It was blue.

The refractive index and film thickness of each of the first to third layers are shown in Table 3.

Table 3

| Coating Layers | Refractive Index | Film Thickness (nm) |
|---|---|---|
| First layer: titania film | 2.3 | 50 |
| Second layer: polystyrene film | 1.5 | 75 |
| Third layer: titania film | 2.3 | 49 |

**EXAMPLE 4**

Intended use: powder for cosmetic material

First layer: titania coating

To 10 g of an acrylic powder (average particle diameter, 1.5 $\mu$m; specific gravity, 1.4) was added 250 ml of ethanol to disperse the particles. The container was heated with an oil bath to keep the temperature of the liquid at 55°C. Thereto was added 4.5 g of titanium isopropoxide. This mixture was stirred. A solution prepared by mixing 30 ml of ethanol with 4.5 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-coated powder $D_1$ was obtained. The titania-coated powder $D_1$ obtained had satisfactory dispersibility and was composed of independent particles. This powder $D_1$ had a spectral reflection curve having a peak wavelength of 545 nm and had a reflectance at the peak wavelength of 31%. It was green.

Second layer: polystyrene coating

To 600 g of distilled water was added 127 g of styrene monomer. While this mixture was heated to 70°C under stirring, sodium lauryl sulfate was added thereto to emulsify the monomer. With this emulsion was mixed 25 g of the titania-coated powder $D_1$. The resultant mixture was agitated at a high speed to sufficiently mix the ingredients. An aqueous ammonium persulfate solution was added thereto in an amount of 10% to initiate a polymerization reaction. The mixture was allowed to react for 4 hours under stirring. After completion of the reaction, the reaction mixture was diluted with 2 liters of distilled water, and the supernatant was discarded by decantation to collect the precipitate. This precipitate was dried on a filter paper to obtain polystyrene-titania-coated powder $D_2$. The polystyrene-titania-coated powder $D_2$ obtained had satisfactory dispersibility and was composed of independent particles.

Third layer: titania coating

To 10 g of the polystyrene-titania-coated powder $D_2$ was added 250 ml of ethanol to disperse the particles. The container was heated with an oil bath to keep the temperature of the liquid at 55°C. Thereto was added 4.5 g of titanium isopropoxide. This fixture was stirred. A solution prepared by mixing 30 ml of ethanol with 4.5 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-polystyrene-coated powder $D_3$ was obtained. The titania-polystyrene-coated powder $D_3$ obtained had satisfactory dispersibility and was composed of independent particles. This powder $D_3$ had a spectral reflection curve having a peak wavelength of 544 nm and had a reflectance at the peak wavelength of 43%. It was green.

Fourth layer: polystyrene coating

To 600 g of distilled water was added 127 g of styrene monomer. While this mixture was heated to 70°C under stirring, sodium lauryl sulfate was added thereto to emulsify the monomer. With this emulsion was mixed 25 g of the titania-polystyrene-coated powder $D_3$. The resultant mixture was agitated at a high speed to sufficiently mix the ingredients. An aqueous ammonium persulfate solution was added thereto in an amount of 10% to initiate a polymerization reaction. The mixture was allowed to react for 4 hours under stirring. After completion of the reaction, the reaction mixture was diluted with 2 liters of distilled water, and the supernatant was discarded by decantation to collect the precipitate. This precipitate was dried on a filter paper to obtain polystyrene-titania-coated powder $D_4$. The polystyrene-titania-coated powder $D_4$ obtained had satisfactory dispersibility and was composed of independent particles.

Fifth layer: titania coating

To 10 g of the polystyrene-titania-coated powder $D_4$ was added 250 ml of ethanol to disperse the particles. The container was heated with an oil bath to keep the temperature of the liquid at 55°C. Thereto was added 4.5 g of titanium isopropoxide. This mixture was stirred. A solution prepared by mixing 30 ml of ethanol with 4.5 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-polystyrene-coated powder D was obtained. The titania-polystyrene-coated powder D obtained had satisfactory dispersibility and was composed of independent particles. This powder D had a spectral reflection curve having a peak wavelength of 552 nm and had a reflectance at the peak wavelength of 58%. It was green. Ultraviolet light having wavelengths of 300 nm and smaller was absorbed by the titania films, and the reflectance in this region was 1% or lower.

The refractive index and film thickness of each of the first to fifth layers are shown in Table 4.

Table 4

| Coating Layers | Refractive Index | Film Thickness (nm) |
|---|---|---|
| First layer: titania film | 2.3 | 59 |
| Second layer: polystyrene film | 1.5 | 92 |
| Third layer: titania film | 2.3 | 59 |
| Fourth layer: polystyrene film | 1.5 | 93 |
| Fifth layer: titania film | 2.3 | 60 |

**EXAMPLE 5**

Intended use: powder for cosmetic material

First layer: titania coating

To 10 g of an acrylic powder (average particle diameter, 1.5 $\mu$m; specific gravity, 1.4) was added 250 ml of ethanol to disperse the particles. The container was heated with an oil bath to keep the temperature of the liquid at 55°C. Thereto was added 5.5 g of titanium isopropoxide. This mixture was stirred. A solution prepared by mixing 30 ml of ethanol with 5.5 g of water was added dropwise to the above mixture over 60 minutes, and the resultant mixture was allowed to react for 2 hours. This reaction mixture was diluted and washed with a sufficient amount of ethanol and then dried with' a vacuum dryer at 180°C for 8 hours. After the drying, titania-coated powder $E_1$ was obtained. The titania-coated powder $E_1$ obtained had satisfactory dispersibility and was composed of independent particles.

Second layer: silver metal coating

Into a silver solution prepared beforehand was dispersed 20 g of the titania-coated powder $E_1$ under stirring. While the particles were kept dispersed by stirring, 600 ml of a reducing solution was added thereto. This mixture was stirred for 30 minutes. As a result, silver metal-titania-coated powder $E_2$ was obtained, which had satisfactory dispersibility. The above silver solution and reducing solution were prepared in the following manners. In preparing the silver solution, 8.75 g of silver nitrate was dissolved in 300 ml of distilled water. Since a precipitate of silver oxide generated, ammonia water (29%) was added until the precipitate changed into complex ions. Subsequently, a solution prepared beforehand by dissolving sodium hydroxide in 300 ml of distilled water was added. Since a precipitate of silver oxide generated again, ammonia water (29%) was added until the precipitate changed into complex ions. Thus, the silver solution was prepared. The reducing solution was prepared by dissolving 45 g of glucose in 1 liter of water, adding 4 g of tartaric acid and dissolving the same, boiling the resultant solution for 5 minutes, cooling the solution, and then adding 100 ml of ethanol.

Third layer: titania coating

Into 250 ml of ethanol was dispersed 10 g of the silver metal-titania-coated powder $E_2$. The container was heated with an oil bath to keep the temperature of the liquid at 55°C. Thereto was added 4.5 g of titanium isopropoxide. This mixture was stirred. A solution prepared by mixing 30 ml of ethanol with 5.5 g of water was added dropwise to the above mixture over 60 minutes, and the resultant mixture was allowed to react for 2 hours. This reaction mixture was diluted and washed with a sufficient amount of ethanol and then dried with a vacuum dryer at 180°C for 8 hours. After the drying, titania-silver metal-coated powder E was obtained. The titania-silver metal-coated powder E obtained had satisfactory dispersibility and was composed of independent particles. This powder had a spectral reflection curve having a bottom wavelength of 585 nm, from which the reflectance increased toward both sides. The powder E had a maximum reflectance of 45% and was purplish-red. In the infrared region, the reflectance increased throughout the range from 780 to 910 nm due to reflection by the silver film, and the maximum reflectance was 60%. In the ultraviolet region, however, ultraviolet light having wavelengths of 300 nm and smaller was absorbed by the silver film and the titania films, and the reflectance in this region was not higher than 5%.

The refractive index and film thickness of each of the first to third layers are shown in Table 5.

Table 5

| Coating Layers | Refractive Index | Film Thickness (nm) |
|---|---|---|
| First layer: titania film | 2.3 | 64 |
| Second layer: silver metal film | 0.1 + 3.5i | 20 |
| Third layer: titania film | 2.3 | 64 |

INDUSTRIAL APPLICABILITY

As described above, according to the present invention, a pigment powder retaining a stable color tone even in long-term storage can be provided which comprises a lightweight base particle and is capable of being colored without using a dye or pigment and with which not only a pigment for monochromatic color inks, e.g., blue, green, or yellow inks, can be obtained by dispersing the pigment powder into a fluid, but also a filler for plastics/papers can be designed.

A monochromatic colored powder, e.g., a blue, green, or yellow powder, can be designed for use as a material for mascaras and eyebrow pencils. Since this powder for use as a material for cosmetics contains no dye or pigment, it is free from the fading caused by the deficiency or denaturation of a dye or pigment and is capable of retaining a bright color over long. It is also possible to design a powder of a multilayer structure which not only serves to color but functions to absorb electromagnetic radiation having harmful wavelengths, such as ultraviolet and infrared rays, and thus prevent the radiation to reach the shin, and which is for use as a material for, e.g., UV-cutting (sunscreen) creams or foundations.

**Claims**

1. A multilayer-coated powder comprising a base particle having a specific gravity of 0.1 to 10.5 and having thereon plural coating layers which are different from each other in refractive index, wherein the base particle is a spherical particle, and wherein each unit of the coating layers has an interference reflection peak or an interference transmission bottom at the same specific wavelength, wherein

the thickness of each unit of the coating layer is determined by fixing a fundamental film thickness thereof which satisfies the following equation (1):

$$N \times d = m \times \lambda/4 \qquad (1)$$

(wherein N represents a complex refractive index, d represents the fundamental film thickness, m represents an integer (natural number), and $\lambda$ represents the wavelength at which the interference reflection peak or interference transmission peak appears, and N is defined by the following equation (2):

$$N = n + i\kappa \qquad (2)$$

(wherein n represents the refractive index of each unit coating layer, i represents complex number, and $\kappa$ represents extinction coefficient)), and correcting the actual thickness of the each unit of the coating layers based on the function of the phase shift caused by the extinction coefficient $\kappa$ of refractive index, the phase shift occurring at film interfaces, and the peak shift attributable to refractive index dispersion and particle shape so that the each unit of the coating layers has an interference reflection peak or an interference transmission bottom at the same specific wavelength.

2. The multilayer-coated powder according to claim 1, wherein at least one of the coating layers is an inorganic metal compound layer.

3. The multilayer-coated powder according to claim 2, wherein the inorganic metal compound layer is a metal oxide film layer.

4. The multilayer-coated powder according to claim 1, wherein at least one of the coating layers is a metal layer or an alloy layer.

5. The multilayer-coated powder according to claim 1, wherein at least one of the coating layers is an organic layer.

6. The multilayer-coated powder according to claim 3, wherein at least one layer of the metal oxide films is formed by hydrolysis of a metal alkoxide.

7. The multilayer-coated powder according to claim 3, wherein at least one layer of the metal oxide films is formed by a reaction of an aqueous solution of a metal salt.

8. A pigment powder, comprising the multilayer-coated powder according to any one of claims 1 to 7.

9. A material for a cosmetic, comprising the multilayer-coated powder according to any one of claims 1 to 7.


**Patentansprüche**

1. Mehrlagig beschichtetes Pulver umfassend ein Basispartikel mit einer spezifischen Schwerkraft von 0,1 bis 10,5 und mit einer Vielzahl von Beschichtungen darauf, welche sich voneinander hinsichtlich des Brechungsindex unterscheiden, wobei das Basispartikel ein sphärisches Partikel ist, und wobei jede Einheit der Beschichtung einen Interferenzreflektionspeak oder einen Interferenztransmissionboden bei der gleichen spezifischen Wellenlänge aufweist, wobei
die Dicke jeder Einheit der Beschichtung bestimmt wird durch das Festlegen einer fundamentalen Filmdicke dieser, welche die folgende Gleichung (1) erfüllt:

$$N \times d = m \times \lambda/4 \qquad (1)$$

(wobei N einen komplexen Brechungsindex darstellt, d die fundamentale Filmdicke darstellt, m eine ganze Zahl (natürliche Zahl) darstellt, und $\lambda$ die Wellenlänge darstellt, bei welcher der Interferenzreflektionspeak oder der Interferenztransmissionspeak auftritt, und n durch die folgende Gleichung (2) definiert ist:

$$N = n + i\kappa \qquad (2)$$

(wobei N den Brechungsindex jeder Einheit der Beschichtung darstellt, i eine komplexe Zahl darstellt und κ den Extinktionskoeffizienten darstellt)) und Korrigieren der tatsächlichen Dicke jeder Einheit der Beschichtungen basierend auf der Funktion der Phasenverschiebung bewirkt durch den Extinktionskoeffizienten κ des Brechungsindex, wobei die Phasenverschiebung an den Filmzwischenflächen auftritt, und die Peakverschiebung der Brechungsindexdispersion und Partikelform zuzuschreiben ist, so dass jede Einheit der Beschichtungen einen Interferenzreflektionspeak oder einen Interferenztransmissionsboden bei der gleichen spezifischen Wellenlänge aufweist.

2. Mehrlagig beschichtetes Pulver nach Anspruch 1, wobei wenigstens eine der Beschichtungen eine anorganische Metallverbindungsschicht ist.

3. Mehrlagig beschichtetes Pulver nach Anspruch 2, wobei die Schicht aus anorganischer Metallverbindung eine Metalloxidfilmschicht ist.

4. Mehrlagig beschichtetes Pulver nach Anspruch 1, wobei wenigstens eine der Beschichtungen eine Metallschicht oder eine Legierungsschicht ist.

5. Mehrlagig beschichtetes Pulver nach Anspruch 1, wobei wenigstens eine der Beschichtungen eine organische Schicht ist.

6. Mehrlagig beschichtetes Pulver nach Anspruch 3, wobei wenigstens eine Schicht der Metalloxidfilme durch Hydrolyse eines Metallalkoxids gebildet ist.

7. Mehrlagig beschichtetes Pulver nach Anspruch 3, wobei wenigstens eine Schicht der Metalloxidfilme durch eine Reaktion einer wässrigen Lösung eines Metallsalzes gebildet ist.

8. Pigmentpulver umfassend das mehrlagig beschichtete Pulver nach einem der Ansprüche 1 bis 7.

9. Material für eine Kosmetik umfassend das mehrlagig beschichtete Pulver gemäß einem der Ansprüche 1 bis 7.

**Revendications**

1. Poudre à revêtement multicouche comprenant une particule de base ayant une densité de 0,1 à 10,5 et ayant sur celle-ci une pluralité de couches de revêtement qui sont différentes les unes des autres en terme d'indice de réfraction, où la particule de base est une particule sphérique, et où chaque unité des couches de revêtement a un pic de réflexion d'interférence ou une vallée de transmission d'interférence à la même longueur d'onde spécifique, où l'épaisseur de chaque unité de la couche de revêtement est déterminée en fixant une épaisseur de film fondamentale de celle-ci qui satisfait à l'équation suivante (1) :

$$N \times d = m \times \lambda/4 \qquad (1)$$

(dans laquelle N représente un indice de réfraction complexe, d représente l'épaisseur de film fondamentale, m représente un entier (nombre naturel), et λ représente la longueur d'onde à laquelle le pic de réflexion d'interférence ou le pic de transmission d'interférence apparaît, et N est défini par l'équation suivante (2) :

$$N = n + i\kappa \qquad\qquad (2)$$

(dans laquelle n représente l'indice de réfraction de chaque couche de revêtement unitaire, i représente un nombre complexe, et $\kappa$ représente un coefficient d'extinction)), et en corrigeant l'épaisseur réelle de chaque unité des couches de revêtement sur la base de la fonction du déphasage causé par le coefficient d'extinction $\kappa$ d'indice de réfraction, le déphasage se produisant aux interfaces de film, et le décalage de pic pouvant être attribué à la dispersion d'indice de réfraction et la forme de particule de sorte que chaque unité des couches de revêtement ait un pic de réflexion d'interférence ou une vallée de transmission d'interférence à la même longueur d'onde spécifique.

2. Poudre à revêtement multicouche selon la revendication 1, dans laquelle au moins une des couches de revêtement est une couche de composé métallique inorganique.

3. Poudre à revêtement multicouche selon la revendication 2, dans laquelle la couche de composé métallique inorganique est une couche de film d'oxyde de métal.

4. Poudre à revêtement multicouche selon la revendication 1, dans laquelle au moins une des couches de revêtement est une couche de métal ou une couche d'alliage.

5. Poudre à revêtement multicouche selon la revendication 1, dans laquelle au moins une des couches de revêtement est une couche organique.

6. Poudre à revêtement multicouche selon la revendication 3, dans laquelle au moins une couche des films d'oxyde de métal est formée par hydrolyse d'un alcoxyde de métal.

7. Poudre à revêtement multicouche selon la revendication 3, dans laquelle au moins une couche des films d'oxyde de métal est formée par une réaction d'une solution aqueuse d'un sel de métal.

8. Poudre de pigment, comprenant la poudre à revêtement multicouche selon l'une quelconque des revendications 1 à 7.

9. Matériau pour un cosmétique, comprenant la poudre à revêtement multicouche selon l'une quelconque des revendications 1 à 7.

FIG. 1

## FIG. 2

REFLECTION INTENSITY

380  VISIBLE LIGHT REGION  780.

↓ INDICATES PEAK POSITION

## FIG. 3

REFLECTION INTENSITY

380  VISIBLE LIGHT REGION  780

↓ INDICATES PEAK POSITION

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6228604 A
- JP 7090310 A
- JP 60169412 A
- JP 2016044 A
- JP 3120351 A
- JP 4168163 A
- JP 8302237 A
- JP 443083 B
- JP 58177911 A

- JP 1158077 A
- JP 3093898 A
- JP 1006093 A
- JP 2029472 A
- GB 2253839 A
- EP 668329 A
- JP 7207251 A
- EP 515928 A